# EUROPÄISCHE PATENTANMELDUNG

(11) **EP 2 336 126 A1**
(43) Veröffentlichungstag der Anmeldung: **22.06.2011**
(21) Anmeldenummer: 10180163.7
(22) Anmeldetag: 29.10.2004
(51) Int. Cl.: C07D 451/10, A61K 31/46, A61P 11/00

(54) **Tiotropiumsalze, Verfahren zu deren Herstellung und Arzneimittelformulierungen diese enthaltend**

(30) Priorität: 03.11.2003 EP 03025075
(62) Teilanmeldung aus: 09153379.4
(71) Anmelder: Boehringer Ingelheim International GmbH, 55216 Ingelheim (DE); Boehringer Ingelheim Pharma GmbH & Co. KG, 55216 Ingelheim am Rhein (DE)
(72) Erfinder: Banholzer, Rolf, 55216 INGELHEIM AM RHEIN (DE); Sieger, Peter, 55216 INGELHEIM AM RHEIN (DE); Pfrengle, Waldemar, 55216 INGELHEIM AM RHEIN (DE)
(74) Vertreter: Hammann, Heinz

(57) **Zusammenfassung**

Die Erfindung betrifft kristallines Tiotropiumjodid, dieses enthaltende Arzneimittelformulierungen sowie deren Verwendung zur Herstellung eines Arzneimittels zur Behandlung von Atemwegserkrankungen, insbesondere zur Behandlung von COPD (chronic obstructive pulmonary disease = chronisch obstruktive Lungenerkrankung) und Asthma.

## Beschreibung

Die Erfindung betrifft ein Verfahren zur Herstellung neuer Tiotropiumsalze, diese neuen Tiotropiumsalze als solche, diese enthaltende Arzneimittelformulierungen sowie deren Verwendung zur Herstellung eines Arzneimittels zur Behandlung von Atemwegserkrankungen, insbesondere zur Behandlung von COPD (chronic obstructive pulmonary disease = chronisch obstruktive Lungenerkrankung) und Asthma.

### Hintergrund der Erfindung

Tiotropiumbromid ist aus der Europäischen Patentanmeldung EP 418 716 A1 bekannt und weist die folgende chemische Struktur auf:

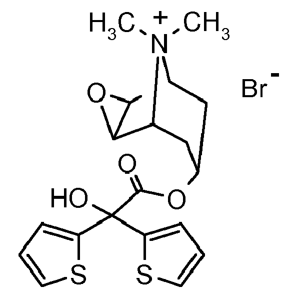

Tiotropiumbromid stellt ein hoch wirksames Anticholinergikum mit lang anhaltender Wirkdauer dar, welches zur Therapie von Atemwegserkrankungen, insbesondere von COPD (chronic obstructive pulmonary disease = chronisch obstruktive Lungenerkrankung) und Asthma Verwendung finden kann. Unter der Bezeichnung Tiotropium ist das freie Ammoniumkation zu verstehen.

Andere Salze des Tiotropiums als das Bromid wurden im Stand der Technik bisher noch nicht explizit beschrieben. Über die in EP 418 716 beschriebene Methodik (vgl. Schema 1) sollten die Halogenide sowie die Alkyl- und Arylsulfonate des Tiotropiums in analoger Art und Weise ebenfalls zugänglich sein. Weitere Salze des Tiotropiums sind mittels dieser Methodik allerdings nicht darstellbar.

Es ist Aufgabe der vorliegenden Erfindung, ein alternatives Syntheseverfahren zur Darstellung von Tiotropiumsalzen bereitzustellen, welches die Synthese weiterer Tiotropiumsalze mittels einer einfachen, universell anwendbaren und schonenden Methodik erlaubt.

### Detaillierte Beschreibung der Erfindung

Die eingangs genannte Aufgabe wird durch das nachstehend beschriebene, erfindungsgemäße Verfahren gelöst.

Die Erfindung betrifft ein Verfahren zur Herstellung neuer Tiotropiumsalze der Formel **1** 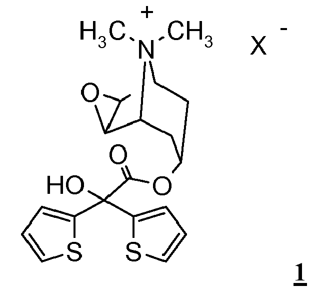 worin X⁻ ein Anion bedeutet,
dadurch gekennzeichnet, dass ein Tiotropiumsalz der Formel **2** 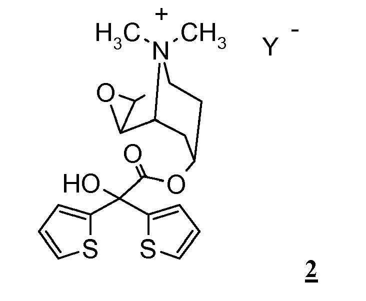 worin
Y⁻ ein von X⁻ verschiedenes Anion ausgewählt aus der Gruppe bestehend aus

Halogenid, C₁-C₁₀-Alkylsulfonat, C₁-C₁₀-Alkylsulfat, C₆-C₁₀-Arylsulfonat bedeutet, mit einer Ionenquelle Kat-X in der Kat für ein Kation steht und X die vorstehend genannten Bedeutungen haben kann, in einem geeigneten Lösemittel umgesetzt wird.

Im erfindungsgemäßen Verfahren werden als Quelle für die Anionen X⁻ die Verbindungen Kat-X eingesetzt. Hierbei handelt es sich um Salze, die neben dem Anion X⁻ ein Kation (Kat) enthalten. Prinzipiell sind sämtliche Salze Kat-X, in denen X die vorstehend genannten Bedeutungen haben kann, für die erfindungsgemäße Umsetzung verwendbar. Bevorzugt sind allerdings solche Salze Kat-X, in denen Kat für ein Kation ausgewählt aus der Reihe der Alkali- oder Erdalkalimetalle steht. Darüber hinaus können erfindungsgemäß bevorzugt auch solche Salze zur Anwendung gelangen, in denen Kat für Ammonium (NH₄⁺) oder auch Tetralkylammonium (N(C₁-C₈-Alkyl)₄⁺ bevorzugt N(C₁-C₄-Alkyl)₄⁺) steht. Besonders bevorzugt sind im erfindungsgemäßen Verfahren solche Verbindungen Kat-X, in denen Kat für Kationen steht, die durch Lithium, Natrium, Kalium, Magnesium, Calcium, Ammonium oder
N(C₁-C₄-Alkyl)₄⁺ gebildet werden. Im Falle mehrwertiger Kationen sind die dadurch gebildeten Salze ((wie z.B. MgI₂) erfindungsgemäß von der Bezeichnung "Kat-X" umfasst, die dementsprechend nicht als auf stöchiometrisch zusammengesetzte Salze beschränkt anzusehen ist.

Von erfindungsgemäß herausragender Bedeutung sind Salze, die durch Natrium, Kalium oder Ammonium, bevorzugt Natrium, Tetrabutylammonium oder Ammonium, bevorzugt Natrium oder Ammonium gebildet werden.

Das erfindungsgemäße Verfahren wird bevorzugt in einem polaren Lösemittel ausgeführt. Besonders bevorzugt gelangen solche Lösemittel zur Anwendung, in denen sowohl das eingesetzte Reagenz Kat-X als auch das entstehende Nebenprodukt Kat-Y löslich sind. Entsprechend geeignete Lösemittel sind dem Fachmann durch einfache Routineexperimente leicht erkennbar. Lösemittel, in denen die Produkt der Formel **1** bei beispielsweise bei Raumtemperatur (etwa 20-25°C) schlechter löslich sind als die Komponenten Kat-X und Kat-Y sind erfindungsgemäß besonders bevorzugt, da sie die Aufarbeitung der Reaktion besonders erleichtern. Bevorzugte Lösemittel sind protische Lösemittel wie Alkohole (beispielsweise Methanol, Ethanol, Isopropanol) und Wasser, bevorzugt Wasser mit pH 2-6 sowie polare organische Lösemittel ausgewählt aus der Gruppe bestehend aus Alkoholen wie beispielsweise Ethylenglycol und Diethylenglycol, Amiden wie beispielsweise Dimethylformamid und N-Methyl-pyrrolidinon, Ethern wie beispielsweise Tetrahydrofuran, Dioxan, Dimethylether und Nitrilen wie beispielsweise Acetonitril. Besonders bevorzugt gelangen als Lösemittel Wasser, Methanol, Ethanol, Isopropanol, Ethylenglycol, Diethylenglycol, Dimethylformamid, N-Methyl-pyrrolidinon, Tetrahydrofuran, Dioxan, Dimethylether oder Acetonitril zur Anwendung, wobei Wasser, insbesondere wässerige Lösungen mit einem pH von etwa 2-6 erfindungsgemäß besonders bevorzugt sind.

Zur Durchführung des erfindungsgemäßen Verfahrens sind, bezogen auf eingesetzte Ausgangsverbindung **2** wenigstens stöchiometrische Mengen des Reagenzes Kat-X erforderlich. Erfindungsgemäß bevorzugt gelangt das Reagenz Kat-X allerdings im Überschuss zur Anwendung. Vorzugsweise werden bezogen auf eingesetzte Verbindung **2** wenigstens 2 Äquivalente, bevorzugt wenigstens 5 Äquivalente, besonders bevorzugt wenigstens 10 Äquivalente, ferner bevorzugt wenigstens 50 Äquivalente Kat-X eingesetzt. Grundsätzlich sind solche Umsetzungen erfindungsgemäß bevorzugt, in denen der Überschuss an Kat-X möglichst groß ist. Zu berücksichtigen ist hierbei je nach Wahl des einzusetzenden Lösemittels die Löslichkeit des Reagenz Kat-X. Erfindungsgemäß besonders bevorzugt gelangen gesättigte Lösungen des Reagenz Kat-X zum Einsatz.

Die erfindungsgemäße Umsetzung erfolgt bevorzugt durch Aufnahme der Verbindung der Formel **2** in mit Kat-X gesättigte Lösungen und Reaktion bei einer Temperatur von wenigstens 0°C bis maximal der Siedetemperatur des verwendeten Lösemittels. Bevorzugt wird die Reaktion allerdings bei weniger als 100°C, besonders bevorzugt bei weniger als 80°C, ferner bevorzugt bei weniger als 60°C durchgeführt. Besonders bevorzugt erfolgt die erfindungsgemäße Umsetzung bei einer Temperatur im Bereich von 10-40°C, bevorzugt bei etwa 20-30°C. Im Vergleich zur Umsetzung bei höheren Temperaturen, können Temperaturen im Bereich von etwa 10-40°C zu verlängerten Reaktionszeiten führen. Dennoch sind Reaktionstemperaturen im Bereich von etwa 10-40°C aufgrund der schonenden Reaktionsbedingungen erfindungsgemäß bevorzugt. Zur Aufarbeitung der Reaktion werden die Verbindungen der Formel **1** abfiltriert und falls erforderlich umkristallisiert.

Die erfindungsgemäßen Umsetzungen können ebenfalls unter Verwendung von im Stand der Technik bekannten Ionenaustauschern durchgeführt werden. Bei besagten Ionenaustauschern handelt es sich um im Stand der Technik bekannte Materialien. Beispielsweise können hierzu Materialien ausgewählt aus der Gruppe bestehend aus Styrol, Styrol-Divenylbenzol (Styrol-DVB) oder auch Polyacryl zum Einsatz gelangen. Besonders bevorzugt werden solche Harze verwendet, die kationische funktionelle Gruppen aufweisen und dadurch mit den vorstehend genannten Anionen X⁻ beladen werden können. Beispielsweise seien genannt Styrol-DVB mit funktionellen Gruppen ausgewählt aus -NMe₃⁺, -NMe₂(CH₂CH₂OH)⁺ oder auch -NH₃⁺. Solche Harze sind im Stand der Technik bekannt und käuflich erhältlich. Durch Einwirken von Kat-X enthaltenen Lösungen, können diese Ionenaustauscherharze mit den entsprechenden Ionen X⁻ beladen werden. Lösungen der Ausgangsverbindungen der Formel 2 in einem der vorstehend genannten Lösemittel können erfindungsgemäß mit den mit X⁻ beladenen Ionenaustauscherharzen in Kontakt gebracht werden. Die nach Abtrennen des Ionenaustauschers oder Durchlaufen entsprechend beladener Ionenaustauschersäulen erhaltenen Lösungen enthalten die Verbindungen der Formel **1**. Diese können daraus in hochreiner Form erhalten werden.

Erfindungsgemäß bevorzugt ist das erfindungsgemäße Verfahren, in dem solche Verbindungen der Formel **2** als Ausgangsprodukt zum Einsatz gelangen, bei denen Y⁻ ein von X⁻ verschiedenes Anion ausgewählt aus der Gruppe bestehend aus Fluorid,
Chlorid, Bromid, Jodid, C₁-C₄-Alkylsulfat, oder
C₁-C₄-Alkylsulfonat, welches am Alkylrest gegebenenfalls ein- oder mehrfach durch Fluor substituiert sein kann, oder
Phenylsulfonat, wobei das Phenylsulfonat am Phenylring gegebenenfalls ein- oder mehrfach durch C₁-C₄-Alkyl, bevorzugt Methyl, substituiert sein kann, bedeutet.

Erfindungsgemäß bevorzugt ist ferner vorstehendes Verfahren, in dem solche Verbindungen der Formel **2** als Ausgangsprodukt zum Einsatz gelangen, bei denen Y⁻ ein von X⁻ verschiedenes Anion ausgewählt aus der Gruppe bestehend aus Fluorid, Chlorid, Bromid, Jodid, Methylsulfat, Ethylsulfat, Methansulfonat, Ethansulfonat, Fluormethansulfonat, Difluormethansulfonat, Trifluormethansulfonat, Phenylsulfonat und Toluolsulfonat bedeutet.

Bevorzugt ist vorstehendes Verfahren, in dem solche Verbindungen der Formel **2** als Ausgangsprodukt zum Einsatz gelangen, bei denen
Y⁻ ein von X⁻ verschiedenes Anion ausgewählt aus der Gruppe bestehend aus Chlorid, Bromid, Jodid, Methylsulfat, Ethylsulfat, Methansulfonat, Trifluormethansulfonat und Toluolsulfonat bedeutet.

Besonders bevorzugt ist vorstehendes Verfahren, in dem solche Verbindungen der Formel **2** als Ausgangsprodukt zum Einsatz gelangen, bei denen
Y⁻ ein von X⁻ verschiedenes Anion ausgewählt aus der Gruppe bestehend aus Bromid, Methylsulfat, Methansulfonat, Trifluormethansulfonat und Toluolsulfonat, bevorzugt Bromid. Methylsulfat oder Methansulfonat bedeutet.

Besonders bevorzugt werden mittels des vorstehend genannten Verfahrens solche Salze **1** erhalten, in denen
X⁻ ein Anion ausgewählt aus der Gruppe bestehend aus Fluorid, Chlorid, Bromid, Jodid, C₁-C₄-Alkylsulfat, Sulfat, Hydrogensulfat, Phosphat, Hydrogenphosphat, Dihydrogenphosphat, Nitrat, Maleat, Acetat, Trifluoracetat, Citrat, Fumarat, Tartrat, Oxalat, Succinat, und Benzoat, oder
C₁-C₄-Alkylsulfonat, welches am Alkylrest gegebenenfalls ein-, zwei oder dreifach durch Fluor substituiert sein kann, oder
Phenylsulfonat, wobei das Phenylsulfonat am Phenylring gegebenenfalls ein- oder mehrfach durch C₁-C₄-Alkyl substituiert sein kann, bedeutet.

Besonders bevorzugt werden mittels des vorstehend genannten Verfahrens ferner solche Salze **1** erhalten, in denen
X⁻ ein Anion ausgewählt aus der Gruppe bestehend aus Fluorid, Chlorid, Jodid, Methylsulfat, Ethylsulfat, Sulfat, Hydrogensulfat, Phosphat, Hydrogenphosphat, Dihydrogenphosphat, Nitrat, Maleat, Acetat, Trifluoracetat, Citrat, Fumarat, Tartrat, Oxalat, Succinat, Benzoat, Methansulfonat, Ethansulfonat, Fluormethansulfonat, Difluormethansulfonat, Trifluormethansulfonat, Phenylsulfonat und Toluolsulfonat bedeutet.

Erfindungsgemäß bevorzugt werden mittels des vorstehend genannten Verfahrens ferner solche Salze **1** erhalten, in denen
X⁻ ausgewählt ist aus Fluorid, Chlorid, Jodid, Nitrat, Maleat, Acetat, Trifluoracetat, Benzoat, Methansulfonat, Trifluormethansulfonat und Toluolsulfonat, wobei bevorzugt solche Salze **1** gemäß dem erfindungsgemäßen Verfahren erhalten werden, in denen X⁻ ausgewählt ist aus Chlorid, Jodid, Acetat, Trifluoracetat und Benzoat, bevorzugt Chlorid und Jodid.

Die vorliegende Erfindung betrifft ferner die Verwendung der Verbindungen der Formel **2** worin Y⁻ die vorstehend genannten Bedeutungen haben kann, als Ausgangsverbindung zur Herstellung der Verbindungen der Formel **1**.

Als C₁-C₁₀Alkyl werden, soweit nicht anders angegeben, verzweigte und unverzweigte Alkylgruppen mit 1 bis 10 Kohlenstoffatomen, bevorzugt 1 bis 4 Kohlenstoffatomen bezeichnet. Beispielsweise werden genannt: Methyl, Ethyl, Propyl oder Butyl. Zur Bezeichnung der Gruppen Methyl, Ethyl, Propyl oder auch Butyl werden gegebenenfalls auch die Abkürzungen Me, Et, Prop oder Bu verwendet. Sofern nicht anders beschrieben, umfassen die Definitionen Propyl und Butyl alle denkbaren isomeren Formen der jeweiligen Reste. So umfasst beispielsweise Propyl n-Propyl und iso-Propyl, Butyl umfasst iso-Butyl, sec. Butyl und tert.-Butyl etc.

Sofern nicht anders angegeben können Alkylreste auch sofern sie Bestandteil anderer Reste sind (z.B. Alkylsulfonat) gegebenenfalls substituiert sein, beispielsweise durch einen oder mehrere Reste ausgewählt aus der Gruppe bestehend aus Fluor, Chlor, Brom, CF₃ Hydroxy oder Methoxy.

Halogen steht im Rahmen der vorliegenden Erfindung für Fluor, Chlor, Brom oder Jod.

Der Begriff C₆-C₁₀-Aryl steht für ein aromatisches Ringsystem mit 6 bis 10 Kohlenstoffatomen. Bevorzugte Arylreste sind Phenyl oder Naphthyl. Diese können gegebenenfalls substituiert sein, beispielsweise durch einen oder mehrere Reste ausgewählt aus der Gruppe bestehend Methyl, Fluor, Chlor, Brom, Hydroxy, CF₃ oder Methoxy.

Die Darstellung der Ausgangsverbindungen der Formel **2** erfolgt beispielsweise in Analogie zu der in der EP-A-418716 offenbarten Methodik. Diese ist im nachstehenden Schema 1 skizziert.

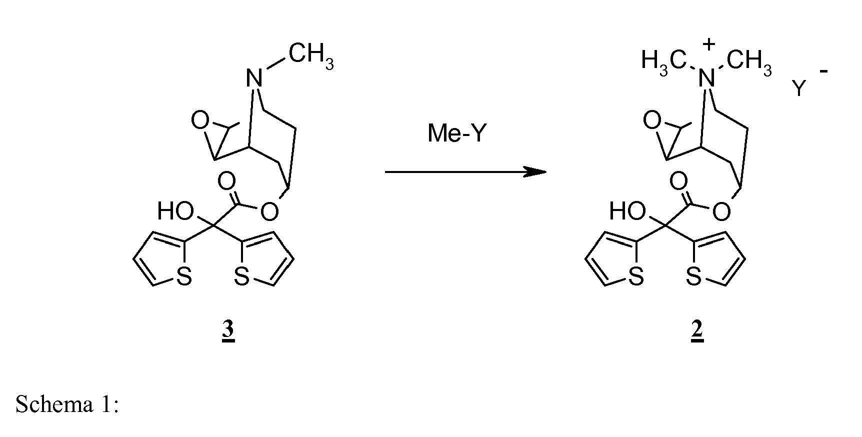

Ausgehend von Scopindithienylglykolsäureester **3** sind die Ausgangsverbindungen **2** durch Umsetzung mit dem Reagenz Me-Y erhältlich.

Im Stand der Technik wurde bisher lediglich die Synthese des Tiotropiumbromids (nach Schema 1) beschrieben. Insofern diejenigen Verbindungen der Formel **2**, in denen Y⁻ eine von Bromid verschiedene Bedeutung hat, neu sind und wie das Tiotropiumbromid als Ausgangsverbindungen in die erfindungsgemäße Synthese zur Herstellung der Verbindungen der Formel **1** eingesetzt werden können, betrifft die vorliegende Erfindung ferner die Ausgangsverbindungen der Formel **2** als solche, in denen Y⁻ bis auf Bromid sämtliche der vorstehend genannten Bedeutungen haben kann, gegebenenfalls in Form ihrer Solvate oder Hydrate.

Beispielsweise werden mittels dieser Vorgehensweise die nachstehenden, noch nicht im Stand der Technik beschriebenen erfindungesgmäß ferner bevorzugten Ausgangsverbindungen der Formel **2** erhalten:
- Di-(2-thienyl)glykolsäurescopinester-methomethansulfonat (Tiotropium-methansulfonat);
- Di-(2-thienyl)glykolsäurescopinester-methomethylsulfat (Tiotropium-methylsulfat).

Insofern diese neuen Verbindungen als Ausgangsverbindungen im erfindungsgemäßen Verfahren zum Einsatz gelangen können, zielt die vorliegende Erfindung besonders bevorzugt ferner auf die vorstehend genannten beiden Verbindungen als solche, gegebenenfalls in form ihrer Solvate oder Hydrate.

Die folgenden Beispiele dienen einer weitergehenden Erläuterung der vorliegenden Erfindung, ohne den Umfang der Erfindung allerdings auf die nachfolgenden beispielhaften Ausführungsformen zu beschränken.

### A. I. Ausgangsmaterialien

### A. 1.1. Tiotropiumbromid:

Tiotropiumbromid ist beispielsweise erhältlich gemäß der in der europäischen

Patentanmeldung EP 418 716 beschriebenen Vorgehensweise.

### A. I.2. Tiotropiummethansulfonat:

75,5 g Di-(2-thienyl)glykolsäurescopinester werden in 750 ml Acetonitril unter leichtem Erwärmen gelöst. Nach Zugabe von 22 ml Methansulfonsäuremethylester wird bei 55°C gerührt. Nach beendeter Reaktion werden etwa 350 ml Lösemittel unter vermindertem Druck abdestilliert. Das Produkt kristallisiert aus und wird abfiltriert. Die Reinigung erfolgt durch Umkristallisation aus Methanol/Aceton.

Ausbeute: 83,35 g weiße Kristalle (74,3 %); Schmp.: 229-231°C (unter Zersetzung).

### A. 1.3. Tiotropiummethylsulfat:

Analog zu der unter Ziffer I.2 beschriebenen Vorgehensweise werden 75,5 g Di-(2-thienyl)glykolsäurescopinester in 750 ml Acetonitril mit 20,9ml Dimethylsulfat umgesetzt. Das auskristallisierte Rohprodukt wird abgetrennt und zur Reinigung aus Methanol umkristallisiert.

Ausbeute: 83,89 g weiße Kristalle (77,5 %); Schmp.: 183-184°C (unter Zersetzung).

### A. II. erfindungsgemäße Synthesebeispiele

### Beispiel 1: Tiotropiumchlorid

1,00g Tiotropiumbromid wird in 100 ml gesättigter NaCl-Lösung (35,8g NaCl/100g E-Wasser) suspendiert und für 14 h bei Raumtemperatur gerührt. Es wird anschließend filtriert und das so gewonnene Produkt erneut für 4 h in 100 ml ges. NaCl- Lösung (35,8g/100g) suspendiert. Das Produkt wird durch Filtration isoliert, getrocknet und anschl. in 15 ml Methanol bei Siedetemperatur aufgenommen. Es wird heiß von Unlöslichem filtriert und das Filtrat auf Raumtemperatur abgekühlt, wobei das Produkt kristallisiert. Ausbeute: 486,2 mg (53,7%); farbloses Kristallpulver; Schmp.: 234°C (Zersetzung);

Anionenbestimmung via HPLC: Chlorid 7,99% (ber.: 8,28%); das Bromid ist nicht mehr detektierbar.

### Beispiel 2: Tiotropiumjodid

5,00g Tiotropiumbromid werden in 50 ml ges. Ammoniumjodid- Lösung (85 g NH₄I / 50g Wasser) suspendiert und für 2 Tage bei Raumtemperatur gerührt. Es wird anschließend filtriert, das so gewonnene Produkt getrocknet und anschließend in 85 ml Methanol bei Siedetemperatur aufgenommen. Es wird heiß von Unlöslichem filtriert und das Filtrat auf Raumtemperatur abgekühlt, wobei das Produkt kristallisiert.

Ausbeute: 4,41g (80%); farbloses Kristallpulver; Schmp. 205°C;

Anionenbestimmung via HPLC: Jodid 24,28% (ber.: 24,43%); das Bromid ist nicht mehr detektierbar.

In analoger Art und Weise werden die erhaltenen Produkte **1** ausgehend von Tiotropiummethylsulfat oder Tiotropiummethansulfonat erhalten.

### A. III. Charakterisierung der erfindungsgemäßen Synthesebeispiele

Die nach obigem Verfahren erhaltenen Verbindungen wurden mit Hilfe der Röntgenpulverbeugung weitergehend charakterisiert. Zur Aufnahme der nachstehend aufgeführten Röntgenpulverdiagramme wurde wie folgt verfahren.

Die Röntgenpulverdiagramme wurden im Rahmen der vorliegenden Erfindung aufgenommen mittels eines Bruker D8 Advanced mit einem OED (= ortsempfindlicher Detektor) (CuK_{α} - Strahlung, λ = 1.5418 Å, 30 kV, 40 mA).

### Beispiel 1: Tiotropiumchlorid

Das gemäß vorstehender Methodik erhaltene Tiotropiumchlorid ist hochkristallin und wird in wasserfreier Form erhalten. Es wurde mit Hilfe der Röntgenpulverbeugung weitergehend untersucht.

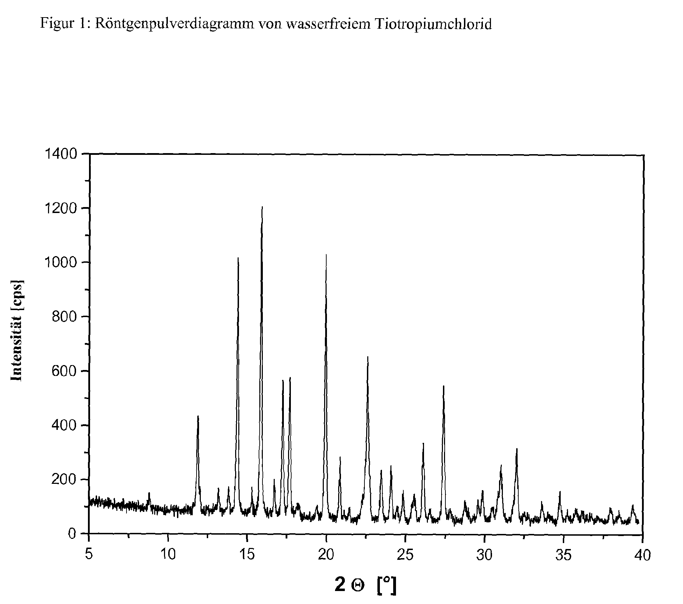

Das für das wasserfreie Tiotropiumchlorid erhaltene Röntgenpulverdiagramm ist in Figur 1 dargestellt.

In nachstehender Tabelle 1 sind die charakteristischen Peaks und normierten Intensitäten aufgelistet.

**Tabelle 1:**

| **2 Θ [°]** | **dₕₖₗ [Å]** | **Intensität [%]** |
|---|---|---|
| 8,83 | 10,01 | 6 |
| 11,87 | 7,45 | 31 |
| 13,2 | 6,7 | 8 |
| 13,82 | 6,4 | 8 |
| 14,39 | 6,15 | 83 |
| 15,29 | 5,79 | 9 |
| 15,87 | 5,58 | 100 |
| 16,72 | 5,3 | 12 |
| 17,24 | 5,14 | 44 |
| 17,68 | 5,01 | 45 |
| 18,22 | 4,86 | 4 |
| 19,38 | 4,58 | 4 |
| 19,95 | 4,45 | 86 |
| 20,85 | 4,26 | 20 |
| 21,45 | 4,14 | 4 |
| 22,59 | 3,93 | 53 |
| 23,47 | 3,79 | 16 |
| 24,09 | 3,69 | 18 |
| 24,48 | 3,63 | 5 |
| 24,84 | 3,58 | 10 |
| 25,56 | 3,48 | 9 |
| 26,12 | 3,41 | 25 |
| 26,53 | 3,36 | 4 |
| 27,41 | 3,25 | 44 |
| 27,83 | 3,2 | 4 |
| 28,76 | 3,1 | 7 |
| 29,59 | 3,02 | 7 |
| 29,86 | 2,99 | 10 |
| 30,49 | 2,93 | 4 |

In vorstehender Tabelle steht der Wert "2 Θ [°]" für den Beugungswinkel in Grad und der Wert dₕₖₗ [Å]" für die bestimmten Gitterebenenabstände in Ä.

Das nach dem erfindungsgemäßen Syntheseverfahren erhaltene Tiotropiumchlorid ist hoch kristallin und eignet sich deshalb besonders gut zur Darstellung von beispielsweise inhalativ zu applizierenden Arzneimittelformulierungen wie Inhalationspulvern oder beispielsweise auch treibgashaltigen Aerosolformulierungen..

Dementsprechend zielt die vorliegende Erfindung ferner auf Tiotropiumchlorid als solches, insbesondere auf kristallines Tiotropiumchlorid, gegebenenfalls in Form seiner Hydrate oder Solvate. Besonders bevorzugt ist dabei ein kristallines Tiotropiumchlorid, welches dadurch gekennzeichnet ist, dass es im Röntgenpulverdiagramm unter anderen die charakteristischen Werte d= 6,15 Å; 5,58 Å; 4,45 Ä und 3,93 Ä aufweist.

Das nach vorstehendem Verfahren erhältliche Tiotropiumchlorid kann durch gezieltes Einwirken von Feuchtigkeit (i.e. Wasserdampf o.ä.) direkt in sein entsprechendes Hydrat überführt werden. Dementsprechend betrifft die vorliegende Erfindung ferner vorstehend genanntes Tiotropiumchlorid in Form seines Hydrats.

### Beispiel 2: Tiotropiumjodid

Das gemäß vorstehender Methodik erhaltene Tiotropiumjodid ist hochkristallin und wird in wasserfreier Form erhalten. Es wurde mit Hilfe der Röntgenpulverbeugung weitergehend untersucht.

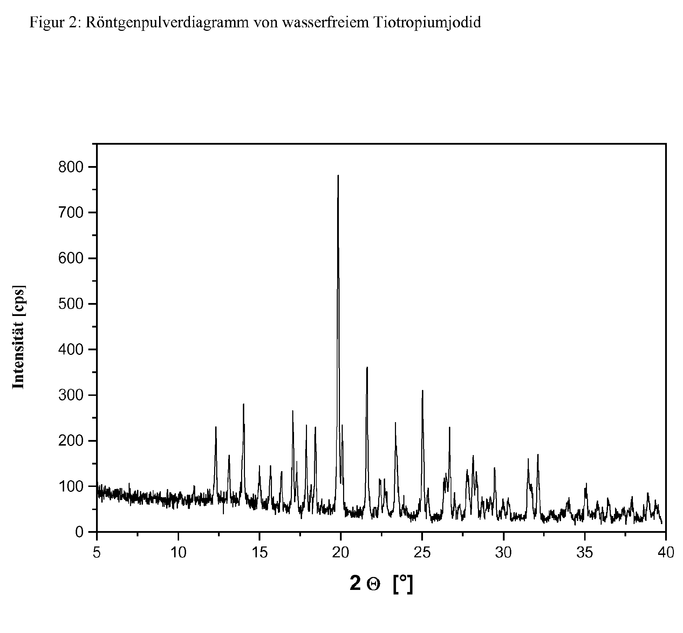

Das für das wasserfreie Tiotropiumjodid erhaltene Röntgenpulverdiagramm ist in Figur 2 dargestellt.

In nachstehender Tabelle 2 sind die charakteristischen Peaks und normierten Intensitäten aufgelistet.

**Tabelle 2:**

| **2 Θ [°]** | **dₕₖₗ [Å]** | **Intensität [%]** |
|---|---|---|
| 11,00 | 8,04 | 5 |
| 12,32 | 7,18 | 23 |
| 13,15 | 6,73 | 15 |
| 14,04 | 6,30 | 31 |
| 15,03 | 5,89 | 13 |
| 15,68 | 5,65 | 13 |
| 16,36 | 5,41 | 11 |
| 17,07 | 5,19 | 30 |
| 17,29 | 5,12 | 15 |
| 17,88 | 4,96 | 26 |
| 18,18 | 4,88 | 8 |
| 18,44 | 4,81 | 25 |
| 19,84 | 4,47 | 100 |
| 20,09 | 4,42 | 26 |
| 21,61 | 4,11 | 44 |
| 22,42 | 3,96 | 11 |
| 22,69 | 3,92 | 11 |
| 22,82 | 3,89 | 7 |
| 23,38 | 3,8 | 28 |
| 25,04 | 3,55 | 38 |
| 25,36 | 3,51 | 9 |
| 26,35 | 3,38 | 11 |
| 26,50 | 3,36 | 13 |
| 26,69 | 3,34 | 27 |
| 26,99 | 3,30 | 8 |
| 27,31 | 3,26 | 4 |
| 27,78 | 3,21 | 15 |
| 28,13 | 3,17 | 19 |
| 28,33 | 3,15 | 14 |
| 28,68 | 3,11 | 7 |
| 28,98 | 3,08 | 6 |
| 29,20 | 3,06 | 7 |
| 29,47 | 3,03 | 16 |
| 29,97 | 2,98 | 5 |
| 30,31 | 2,95 | 6 |
| 31,54 | 2,83 | 18 |
| 32,11 | 2,78 | 20 |

In vorstehender Tabelle steht der Wert "2 Θ [°]" für den Beugungswinkel in Grad und der Wert dₕₖₗ [Å]" für die bestimmten Gitterebenenabstände in Ä.

Das nach dem erfindungsgemäßen Syntheseverfahren erhaltene Tiotropiumjodid ist hoch kristallin und eignet sich deshalb besonders gut zur Darstellung von beispielsweise inhalativ zu applizierenden Arzneimittelformulierungen wie Inhalationspulvern oder beispielsweise auch treibgashaltigen Aerosolformulierungen.

Dementsprechend zielt die vorliegende Erfindung ferner auf Tiotropiumjodid als solches, insbesondere auf kristallines Tiotropiumjodid, gegebenenfalls in Form seiner Hydrate oder Solvate. Besonders bevorzugt ist dabei das erfindungsgemäße wasserfreie kristalline Tiotropiumjodid, welches dadurch gekennzeichnet ist, dass es im Röntgenpulverdiagramm unter anderen die charakteristischen Werte d= 6,30 Å; 5,19 Å; 4,47 Å; 4,11 Ä und 3,55 Ä aufweist.

### B. Arzneimittelformulierungen

Die vorliegende Erfindung betrifft ferner neue Arzneimittelformulierungen, die die vorstehend genannten neuen Tiotropiumsalze Tiotropiumchlorid oder Tiotropiumjodid enthalten. Tiotropiumchlorid und Tiotropiumjodid werden bevorzugt inhalativ appliziert. Hierzu kommen inhalativ applizierbare Pulverformulierungen, treibgashaltige Aerosolformulierungen oder treibgasfreie Inhalationslösungen in Betracht.

### B.1. Inhalationspulver

Die vorliegende Erfindung betrifft ferner Inhalationspulver enthaltend 0,001 bis 3 % Tiotropium in Form des erfindungsgemäßen Tiotropiumchlorids oder Tiotropiumjodids im

Gemisch mit einem physiologisch unbedenklichen Hilfsstoff. Unter Tiotropium ist dabei das Ammoniumkation zu verstehen.

Erfindungsgemäß bevorzugt sind Inhalationspulver, die 0,01 bis 2 % Tiotropium enthalten. Besonders bevorzugte Inhalationspulver enthalten Tiotropium in einer Menge von etwa 0,03 bis 1 %, bevorzugt 0,05 bis 0,6 %, besonders bevorzugt 0,06 bis 0,3 %. Erfindungsgemäß von besonderer Bedeutung sind schließlich Inhalationspulver, die etwa 0,08 bis 0,22 % Tiotropium enthalten.

Vorstehend genannte Anteile an Tiotropium sind bezogen auf die Menge enthaltenen Tiotropiumkations. Die erfindungsgemäßen Inhalationspulver enthalten Tiotropium in form des erfindungsgemäßen Tiotropiumchlorids oder Tiotropiumjodids.

Die Hilfsstoffe, die im Sinne der vorliegenden Erfindung zur Anwendung gelangen, werden durch geeignetes Mahlen und/oder Sieben nach gängigen, im Stand der Technik bekannten Verfahren hergestellt. Gegebenenfalls handelt es sich bei den erfindungsgemäß zum Einsatz gelangenden Hilfsstoffen auch um Hilfsstoffgemische, die durch Mischen von Hilfsstofffraktionen unterschiedlicher mittlerer Teilchengröße erhalten werden.

Als physiologisch unbedenkliche Hilfsstoffe, die zur Darstellung der im Rahmen der erfindungsgemäßen Inhaletten zur Anwendung gelangenden Inhalationspulver Verwendung finden können seien beispielsweise genannt Monosaccharide (z.B. Glucose, Fructose, Arabinose), Disaccharide (z.B. Lactose, Saccharose, Maltose, Trehalose), Oligo-und Polysaccharide (z.B. Dextrane, Dextrine, Maltodextrin, Stärke, Cellulose), Polyalkohole (z.B. Sorbit, Mannit, Xylit), Cyclodextrine (z. B. α-Cyclodextrin, β-Cyclodextrin, χ-Cyclodextrin, Methyl-β-Cyclodextrin, Hydroxypropyl-β-Cyclodextrin), Aminosäuren (z.B. Argininhydrochlorid) oder auch Salze (z.B. Natriumchlorid, Calciumcarbonat), oder Mischungen davon Bevorzugt gelangen Mono- oder Disaccharide zur Anwendung, wobei die Verwendung von Lactose oder Glucose, insbesondere, aber nicht ausschließlich in Form ihrer Hydrate, bevorzugt ist. Als besonders bevorzugt im Sinne der Erfindung gelangt als Hilfsstoff Lactose, höchst bevorzugt Lactosemonohydrat zur Anwendung.

Die Hilfsstoffe weisen im Rahmen der erfindungsgemäßen Inhalationspulver eine maximale mittlere Teilchengröße von bis zu 250 µm, bevorzugt zwischen 10 und 150µm besonders bevorzugt zwischen 15 und 80 µm auf. Gegebenenfalls kann es sinnvoll erscheinen, den vorstehend genannten Hilfsstoffen feinere Hilfsstofffraktionen mit einer mittleren Teilchengröße von 1 bis 9µm beizumischen. Letztgenannte feinere Hilfsstoffe sind ebenfalls ausgewählt aus der vorstehend genannten Gruppe an einsetzbaren Hilfsstoffen. Die Bestimmung der mittleren Teilchengröße kann nach im Stand der Technik bekannten Verfahren erfolgen (siehe beispielsweise WO 02/30389, Abschnitte A und C). Schließlich wird zur Herstellung der erfindungsgemäßen Inhalationspulver mikronisiertes Tiotropiumchlorid oder Tiotropiumjodid, welches vorzugsweise durch eine mittlere Teilchengröße von 0,5 bis 10µm, besonders bevorzugt von 1 bis 5µm gekennzeichnet ist, der Hilfsstoffmischung beigemischt. Die Bestimmung der mittleren Teilchengröße kann nach im Stand der Technik bekannten Verfahren erfolgen (siehe beispielsweise WO 02/30389, Abschnitt B). Verfahren zum Mahlen und Mikronisieren von Wirkstoffen sind im Stand der Technik bekannt.

Wird als Hilfsstoff keine spezifisch hergestellte Hilfsstoffmischung verwendet, gelangen besonders bevorzugt solche Hilfsstoffe zur Anwendung, die eine mittlere Teilchengröße von 10-50 µm und einen 10 %-Feinanteil aufweisen.

Dabei wird unter der mittleren Teilchengröße im hier verwendeten Sinne der 50 % - Wert aus der Volumenverteilung gemessen mit einem Laserdiffraktometer nach der Trockendispersionsmethode verstanden. Die Bestimmung der mittleren Teilchengröße kann nach im Stand der Technik bekannten Verfahren erfolgen (siehe beispielsweise WO 02/30389, Abschnitte A und C). Analog ist unter dem 10 % - Feinanteil im hier verwendeten Sinne der 10 % - Wert aus der mit einem Laserdiffraktometer gemessenen Volumenverteilung zu verstehen. Mit anderen Worten steht im Sinne der vorliegenden Erfindung der 10%-Feinanteil-Wert für die Teilchengröße, unterhalb derer 10% der Teilchenmenge liegt (bezogen auf Volumenverteilung).

Bei den im Rahmen der vorliegenden Erfindung genannten prozentualen Angaben, handelt es sich stets um Gewichtsprozent, soweit nichts Gegenteiliges spezifisch hervorgehoben wird.

In besonders bevorzugten Inhalationspulvern ist der Hilfsstoff durch eine mittlere Teilchengröße von 12 bis 35 µm, besonders bevorzugt von 13 bis 30 µm gekennzeichnet. Ferner sind insbesondere solche Inhalationspulver besonders bevorzugt, in denen der 10 %-Feinanteil etwa 1 bis 4 µm, bevorzugt etwa 1,5 bis 3 µm beträgt.

Die erfindungsgemäßen Inhalationspulver sind entsprechend der der vorliegenden Erfindung zugrunde liegenden Aufgabe gekennzeichnet durch ein hohes Maß an Homogenität im Sinne der Einzeldosierungsgenauigkeit. Diese liegt in einem Bereich von < 8 % , bevorzugt < 6 % , besonders bevorzugt < 4 %.

Nach Einwaage der Ausgangsmaterialien erfolgt die Herstellung der Inhalationspulver aus dem Hilfsstoff und dem Wirkstoff unter Verwendung von im Stand der Technik bekannten Verfahren an. Hierbei sei beispielsweise auf die Offenbarung der WO 02/30390 verwiesen. Die erfindungsgemäßen Inhalationspulver sind dementsprechend beispielsweise gemäß der nachfolgend beschriebenen Vorgehensweise erhältlich. Bei den nachstehend beschriebenen Herstellverfahren werden die genannten Komponenten in den Gewichtsanteilen eingesetzt, wie sie in den zuvor beschriebenen Zusammensetzungen der Inhalationspulver beschrieben wurden.

Zunächst werden der Hilfsstoff und der Wirkstoff in einen geeigneten Mischbehälter eingebracht. Der verwendete Wirkstoff weist eine mittlere Teilchengröße von 0,5 bis 10 µm, vorzugsweise von 1 bis 6 µm, besonders bevorzugt von 2 bis 5 µm auf. Die Zugabe des Wirkstoffs und des Hilfsstoffs erfolgt vorzugsweise über ein Sieb oder einen Siebgranulator mit einer Maschenweite von 0,1 bis 2 mm, besonders bevorzugt 0,3 bis 1 mm, höchst bevorzugt 0,3 bis 0,6 mm. Vorzugsweise wird der Hilfsstoff vorgelegt und anschließend der Wirkstoff in den Mischbehälter eingebracht. Bevorzugt erfolgt bei diesem Mischverfahren die Zugabe der beiden Komponenten portionsweise. Besonders bevorzugt ist das abwechselnde, schichtweise Einsieben der beiden Komponenten. Der Mischvorgang des Hilfsstoffs mit dem Wirkstoff kann bereits während der Zugabe der beiden Komponenten erfolgen. Vorzugsweise wird allerdings erst nach dem schichtweisen Einsieben der beiden Bestandteile gemischt.

Die vorliegende Erfindung betrifft ferner die Verwendung der erfindungsgemäßen Inhalationspulver zur Herstellung eines Arzneimittels zur Behandlung von Atemwegserkrankungen, insbesondere zur Behandlung von COPD und/oder Asthma.

Die erfindungsgemäßen Inhalationspulver können beispielsweise mittels Inhalatoren appliziert werden, die eine einzelne Dosis aus einem Vorrat mittels einer Messkammer (z.B. gemäß US 4570630A) oder über andere apparative Vorrichtungen (z.B. gemäß DE 36 25 685 A) dosieren. Vorzugsweise werden die erfindungsgemäßen Inhalationspulver allerdings in Kapseln abgefüllt (zu so genannten Inhaletten), die in Inhalatoren wie beispielsweise in der WO 94/28958 beschrieben, zur Anwendung gelangen.

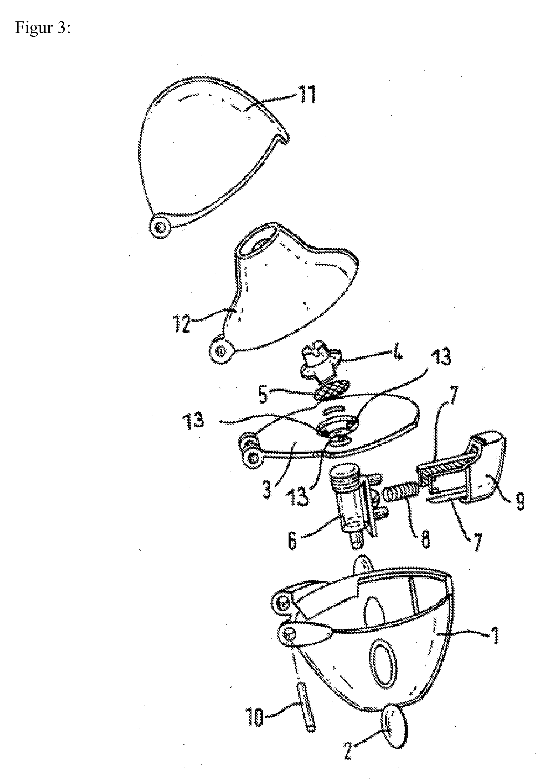

Besonders bevorzugt werden die das erfindungsgemäße Inhalationspulver enthaltenden Kapseln mit einem Inhalator appliziert, wie er in Figur 3 dargestellt ist.

Dieser Inhalator ist gekennzeichnet durch ein Gehäuse 1, enthaltend zwei Fenster 2, ein Deck 3, in dem sich Lufteinlassöffnungen befinden und welches mit einem über ein Siebgehäuse 4 befestigten Sieb 5 versehen ist, eine mit Deck 3 verbundene Inhalationskammer 6, an der ein mit zwei geschliffenen Nadeln 7 versehener, gegen eine Feder 8 beweglicher Drücker 9 vorgesehen ist, ein über eine Achse 10 klappbar mit dem Gehäuse 1, dem Deck 3 und einer Kappe 11 verbundenes Mundstück 12, sowie Luftdurchlasslöcher 13 zur Einstellung des Strömungswiderstandes.

Die vorliegende Erfindung betrifft ferner die Verwendung der erfindungsgemäßen Inhalationspulver zur Herstellung eines Arzneimittels zur Behandlung von Atemwegserkrankungen, insbesondere zur Behandlung von COPD und/oder Asthma, dadurch gekennzeichnet, dass der vorstehend beschriebene, in Figur 3 dargestellte Inhalator zur Anwendung gelangt.

Für die Anwendung der erfindungsgemäßen Inhalationspulver mittels pulverhaltiger Kapseln werden besonders bevorzugt solche Kapseln verwendet, deren Material ausgewählt ist aus der Gruppe der synthetischen Kunststoffe, besonders bevorzugt ausgewählt aus der Gruppe bestehend aus Polyethylen, Polycarbonat, Polyester, Polypropylen und Polyethylenterephthalat. Besonders bevorzugt sind als synthetische Kunststoffmaterialien Polyethylen, Polycarbonat oder Polyethylenterephthalat. Wird Polyethylen als eines der erfindungsgemäß besonders bevorzugten Kapselmaterialien verwendet, gelangt vorzugsweise Polyethylen mit einer Dichte zwischen 900 und 1000 kg/m³, bevorzugt von 940 - 980 kg/m³, besonders bevorzugt von etwa 960 - 970 kg/m³ (high-density Polyethylen) zur Anwendung.

Die synthetischen Kunststoffe im Sinne der Erfindung können vielseitig mittels dem im Stand der Technik bekannten Herstellverfahren verarbeitet werden. Bevorzugt im Sinne der Erfindung wird die spritzgusstechnische Verarbeitung der Kunststoffe. Besonders bevorzugt wird die Spritzgusstechnik unter Verzicht auf die Verwendung von Formtrennmitteln. Dieses Herstellverfahren ist wohldefiniert und durch eine besonders gute Reproduzierbarkeit gekennzeichnet.

Ein weiterer Aspekt der vorliegenden Erfindung betrifft vorstehend genannte Kapseln, die vorstehend genanntes erfindungsgemäßes Inhalationspulver enthalten. Diese Kapseln können etwa 1 bis 20 mg, bevorzugt etwa 3 bis 15 mg, besonders bevorzugt etwa 4 bis 12 mg Inhalationspulver enthalten. Erfindungsgemäß bevorzugte Formulierungen enthalten 4 bis 6 mg Inhalationspulver. Von erfindungsgemäß gleichrangiger Bedeutung sind Inhaltionskapseln, die die erfindungsgemäßen Formulierungen in einer Menge von 8 bis 12 mg enthalten.

Ferner betrifft die vorliegende Erfindung ein Inhalationskit, bestehend aus einer oder mehrerer der vorstehend beschriebenen, durch einen Gehalt an erfindungsgemäßem Inhalationspulver gekennzeichneten Kapseln in Verbindung mit dem Inhalator gemäß Figur 3.

Die vorliegende Erfindung betrifft ferner die Verwendung der vorstehend genannten, durch einen Gehalt an erfindungsgemäßem Inhalationspulver gekennzeichneten Kapseln, zur Herstellung eines Arzneimittels zur Behandlung von Atemwegserkrankungen, insbesondere zur Behandlung von COPD und/oder Asthma.

Die Darstellung von befüllten Kapseln, die die erfindungsgemäßen Inhalationspulver enthalten, erfolgt nach im Stand der Technik bekannten Verfahren durch Befüllung der leeren Kapseln mit den erfindungsgemäßen Inhalationspulvern.

### B.1.1. Beispiele für erfindungsgemäße Inhalationspulver

Die folgenden Beispiele dienen einer weitergehenden Erläuterung der vorliegenden Erfindung, ohne den Umfang der Erfindung allerdings auf die nachfolgenden beispielhaften Ausführungsformen zu beschränken.

### B.1.1.1. Ausgangsmaterialien

### Wirkstoff

Für die Herstellung der erfindungsgemäßen Inhalationspulver werden das erfindungsgemäße Tiotropiumchlorid oder Tiotropiumjodid verwendet. Die Mikronisierung dieser Wirkstoffe erfolgt in Analogie zu im Stand der Technik bekannten Verfahren (siehe beispielsweise WO 03/078429 A1).

### Hilfsstoff:

In den nachfolgenden Beispielen wird als Hilfsstoff Lactosemonohydrat verwendet. Dieser kann beispielsweise von der Firma Borculo Domo Ingredients, Borculo/NL unter der Produktbezeichnung *Lactochem Extra Fine Powder* bezogen werden. Die erfindungsgemäßen Spezifikationen für die Teilchengröße werden von dieser Lactosequalität erfüllt.

### B.1.1.2. Darstellung der erfindungsgemäßen Pulverformulierungen:

### I) Apparatives

Zur Herstellung der Inhalationspulver können beispielsweise die folgenden Maschinen und Geräte Verwendung finden:
Mischbehälter bzw. Pulvermischer: Turbulamischer 2 L, Typ 2C; Hersteller Willy A. Bachofen AG, CH-4500 Basel
Handsieb: 0,135 mm Maschenweite

Die Befüllung der leeren Inhaltionskapseln mittels Tiotropium-haltigen Inhaltionspulver kann händisch oder maschinell erfolgen. Es können nachstehende Geräte verwendet werden.

### Kapselfüllmaschine:

MG2, Typ G100, Hersteller: MG2 S.r.1, 1-40065 Pian di Macina di Pianoro (BO), Italien

### Formulierungsbeispiel 1:

### Pulvermischung :

Zur Herstellung der Pulvermischung werden 299,39 g Hilfsstoff und 0,61 g mikronisiertes Tiotropiumchlorid (oder Tiotropiumjodid) eingesetzt. In den daraus erhaltenen 300 g Inhalationspulver beträgt der Wirkstoffanteil bezogen auf Tiotropium 0,19 % im Falle des Tiotropiumchlorids und 0,15 % im Falle des Tiotropiumjodids.

Über ein Handsieb mit einer Maschenweite von 0,315 mm werden in einen geeigneten Mischbehälter ca. 40-45 g Hilfsstoff vorgelegt. Anschließend werden abwechselnd Tiotropiumchlorid (oder Tiotropiumjodid) in Portionen von ca. 90-110 mg und Hilfsstoff in Portionen von etwa 40-45 g schichtweise eingesiebt. Die Zugabe des Hilfsstoffs und des Wirkstoffs erfolgt in 7 bzw. 6 Schichten.

Die eingesiebten Bestandteile werden anschließend gemischt (Mischen: 900 Umdrehungen). Die Endmischung wird noch zweimal über ein Handsieb gegeben und anschließend jeweils gemischt (Mischen: 900 Umdrehungen).

Gemäß der in Beispiel 1 beschriebenen Vorgehensweise können solche Inhalationspulver erhalten werden, die nach Befüllung der entsprechenden Kunststoffkapseln beispielsweise zu den nachstehenden Inhalationskapseln führen:

### Formulierungsbeispiel 2:

| | |
|---|---|
| Tiotropiumchlorid: | 0,0113 mg |
| Lactose Monohydrat: | 5,4887 mg |
| Polyethylen-Kapseln: | 100,0 mg |
| Total: | 105,5 mg |

### Formulierungsbeispiel 3:

| | |
|---|---|
| Tiotropiumchlorid: | 0,0113 mg |
| Lactose Monohydrat*): | 5,4887 mg |
| Polyethylen-Kapseln: | 100,0 mg |
| Total: | 105,5 mg |

| | |
|---|---|
| *) die Lactose enthält 5% spezifisch zugesetzten Feinanteil an mikronisiertem Lactosemonohydrat mit einer mittleren Teilchengröße von etwa 4µm. | |

### Formulierungsbeispiel 4:

| | |
|---|---|
| Tiotropiumjodid: | 0,0113 mg |
| Lactose Monohydrat: | 5,4887 mg |
| Polyethylen-Kapseln: | 100,0 mg |
| Total: | 105,5 mg |

### Formulierungsbeispiel 5:

| | |
|---|---|
| Tiotropiumjodid: | 0,0225 mg |
| Lactose Monohydrat: | 5,4775 mg |
| Polyethylen-Kapseln: | 100,0 mg |
| Total: | 105,5 mg |

### Formulierungsbeispiel 6:

| | |
|---|---|
| Tiotropiumchlorid: | 0,0056 mg |
| Lactose Monohydrat: | 5,4944 mg |
| Polyethylen-Kapseln: | 100,0 mg |
| Total: | 105,5 mg |

### Formulierungsbeispiel 7:

| | |
|---|---|
| Tiotropiumchlorid: | 0,0056 mg |
| Lactose Monohydrat^{*)}: | 5,4944 mg |
| Polyethylen-Kapseln: | 100,0 mg |
| Total: | 105,5 mg |

| | |
|---|---|
| *) die Lactose enthält 5% spezifisch zugesetzten Feinanteil an mikronisiertem Lactosemonohydrat mit einer mittleren Teilchengröße von etwa 4µm. | |

### Formulierungsbeispiel 8:

| | |
|---|---|
| Tiotropiumjodid: | 0,0113 mg |
| Lactose Monohydrat^{*)}: | 9,9887 mg |
| Polyethylen-Kapseln: | 100,0 mg |
| Total: | 110,0 mg |

| | |
|---|---|
| *) die Lactose enthält 5% spezifisch zugesetzten Feinanteil an mikronisiertem Lactosemonohydrat mit einer mittleren Teilchengröße von etwa 4µm. | |

### Formulierungsbeispiel 9:

| | |
|---|---|
| Tiotropiumjodid: | 0,0225 mg |
| Lactose Monohydrat^{*)}: | 9,9775 mg |
| Polyethylen-Kapseln: | 100,0 mg |
| Total: | 110,0 mg |

| | |
|---|---|
| *) die Lactose enthält 5% spezifisch zugesetzten Feinanteil an mikronisiertem Lactosemonohydrat mit einer mittleren Teilchengröße von etwa 4µm. | |

### B.2. Treibgashaltige Inhalationsaerosole

Die neuen Tiotropiumsalze Tiotropiumchlorid oder Tiotropiumjodid können gegebenenfalls auch in Form von treibgashaltigen Inhalationsaerosolen appliziert werden. Hierzu können Aerosollösungsformulierungen oder Aerosolsuspensionsformulierungen zum Einsatz gelangen.

### B.2.1. Aerosol-lösungsformulierungen

Die Bezeichnung Aerosollösungsformulierung steht dabei für pharmazeutische Formulierungen in denen das Tiotropiumchlorid oder -jodid sowie gegebenenfalls zum Einsatz gelangende Hilfsstoffe vollständig gelöst sind.

Die vorliegende Erfindung stellt Tiotropiumchlorid und -jodid enthaltende Aerosolformulierungen bereit, die neben einem der vorstehenden Tiotropiumsalze ein HFA Treibmittel, ein Cosolvens und eine anorganische oder organische Säure enthalten und die ferner dadurch gekennzeichnet sind, dass die Konzentration der Säure so bemessen ist, dass sie in wässeriger Lösung einem pH im Bereich range von 2.5 - 4.5 entspricht.

Die vorstehend genannten Aerosollösungsformulierungen sind durch eine besonders hohe Stabilität gekennzeichnet.

Bevorzugte Aerosollösungsformulierungen sind dadruch gekennzeichnet, dass die Konzentration der Säure so bemessen ist, dass sie in wässeriger Lösung einem pH im Bereich range 3.0 - 4.3, besonders bevorzugt 3.5 - 4.0 entspricht.

Gegebenenfalls können die erfindungsgemäßen Aerosollösungsformulierungen ferner eine geringe Menge Wasser (vorzugsweise bis zu 5%, besonders bevorzugt bis zu 3%, ferner bevorzugt bis zu 2 %) enthalten.

Die erfindungsgemäßen Aerosollösungsformulierungen enthalten vorzugsweise eine solche Menge an Tiotropiumchlorid oder Tiotropiumjodid, dass der Anteil an enthaltenem Tiotropium-kation zwischen 0,00008 und 0,4 %, bevorzugt zwischen 0,0004 und 0.16 %, besonders bevorzugt zwischen 0,0008 und 0.08 % beträgt.

Geeignet als HFA-Treibmittel sind im Rahmen der Aerosollösungsformulierungen solche, die mit den verwendeten Co-solventien eine homogene Treibmittelformulierung bilden, in der eine therapeutisch wirksame Menge des Tiotropiumchlorids oder -jodids gelöst werden kann. Erfindungsgemäß bevorzugt gelangen als HFA-Treibmittel Treibmittel ausgewähltaus der Gruppe bestehend aus 1,1,1,2-Tetrafluoroethan (HFA-134(a)), 1,1,1,2,3,3,3,-Heptafluoropropan (HFA-227), HFA-32 (Difluoromethan), HFA-143(a) (1,1,1-Trifluoroethan), HFA-134 (1,1,2,2-tetrafluoroethane) und HFA-152a (1,1-Difluoroethan zur Anwendung. HFA-134(a) und HFA-227 sind erfindungsgemäß besonders bevorzugt, wobei HFA-134(a) erfindungsgemäß besondere Bedeutung zukommt. Neben den vorstehend genannten HFA-Treibmitteln können auch nichthalogenierte Treibgase allein oder im Gemisch mit einem oder mehreren der vorstehend genannten HFA-Treibmittel zum Einsatz gelangen. Beispiele für solche nichthalogenierten Treibmittel sind gesättigte Kohlenwasserstoffe wie beispielsweise n-Propan, n-Butan oder Isobutane, oder auch Ether wie beispielsweise Diethylether.

Als Säuren können erfindungsgemäß organische oder anorganische Säuren eingesetzt werden. Anorganische Säuren sind im Rahmen der vorliegenden Erfindung beispielsweise ausgewählt aus der Gruppe bestehend aus Salzsäure, Schwefelsäure, Salpetersäure oder Phosphorsäure, wobei erfindungsgemäß der einsatz von Slaz- oder Schwefelsäure, besonders der von Salzsäure bevorzugt ist. Organische Säuren sind im Rahmen der vorliegenden Erfindung beispielsweise ausgewählt aus der Gruppe bestehend aus Ascorbinsäure, Zitronensäure, Milchsäure, Maleinsäure, Benzoesäure oder Weinsäure, wobei Ascorbinsäure und Zitronensäure erfindungsgemäß bevorzugt sind.

Die erfindungsgemäßen Aerosollösungsformulierungen können in analogie zu im Stand der Technik bekannten Verfahren erhalten werden.

Gegebenenfalls können pharmazeutisch verträgliche Hilfsstoffe in den erfindungsgemäßen Aerosollösungsformulierungen enthalten sein. Beispielsweise können lösliche oberflächenaktive Mittel und Schmiermittel zum Einsatz gelangen. als Beispiele für solche löslichen oberflächenaktiven Mittel und Schmiermittel seien genannt Sorbitantrioleat, Lecithin oder auch Isopropylmyristat. Als Hilfsstoffe können darüberhinaus Antioxidantien (beispielsweise Ascorbinsäure oderTocopherol), geschmacksmaskierende Agentien (beispielsweise Menthol, Süßungsmittel und künstliche oder natürliche Aromastoffe) enthalten sein.

Beispiele für erfindungsgemäß einsetzbare Cosolventien sind Alkohole (beispielsweise Ethanol, Isopropanol und Benzylakohol), Glykole (beispielsweise Propylenglykol, Polyethylenglykole, Polypropylenglykol, Glykolether, Blockcopolymere des Oxyethylens und Oxypropylens) oder auch weitere Substanzen wie beispielsweise Glycerol, Polyoxyethylenalkohole, Polyoxtethylenfettsäureester und Glykofurole (wie beispielsweise Glykofurol 75). Ein erfindungsgemäß bevorzugtes Cosolvens ist Ethanol.

Die Menge an in die erfindungsgemäßen Formulierungen einsetzbarem Cosolvens liegt vorzugsweise im Bereich von 5-50 %, bevorzugt 10 - 40 %, besonders bevorzugt 15 - 30 % bezogen auf die Gesamtformulierung.

Sofern nicht gegenteilig angegeben, sind prozentuale Angaben im Rahmen der vorliegenden Erfindung als Gewichtsprozent zu verstehen.

Die erfindungsgemäßen Formulierungen können, wie vorstehend bereits erwähnt, geringe Mengen Wasser enthalten. Ein bevorzugter Aspekt der vorliegenden Erfindung betrifft Formulierungen, in denen der Wasseranteil bei bis zu 5%, besonders bevorzugt bis zu 3%, ferner bevorzugt bis zu 2 % liegt.

Ein anderer Aspekt der vorliegenden Erfindung betrifft Aerosollösungsformulierungen, die kein Wasser enthalten. In diesen Formulierungen liegt die Menge an cosolvens vorzugsweise im Bereich von 20 - 50%, bevorzugt im Bereich von 30 - 40%.

Die erfindungsgemäßen formulierungen können mittels im Stand der Technik bekannten Inhalatoren (pMDIs = pressurized metered dose inhalers) appliziert werden.

Die vorliegende Erfindung betrifft ferner die Verwendung der vorstehend genannten, durch einen Gehalt an erfindungsgemäßem Tiotropiumchlorid oder -jodid gekennzeichneten Aerosollöungsformulierungen zur Herstellung eines Arzneimittels zur Behandlung von Atemwegserkrankungen, insbesondere zur Behandlung von COPD und/oder Asthma.

Die folgenden Beispiele dienen einer weitergehenden Erläuterung der vorliegenden Erfindung, ohne den Umfang der Erfindung allerdings auf die nachfolgenden beispielhaften Ausführungsformen zu beschränken.

### B.2.1.1 Beispiele für Aerosollösungsformulierungen

### Formulierungsbeispiel 10:

| **Bestandteile** | **Konzentration [% w/w]** |
|---|---|
| Tiotropiumjodid | 0,01 |
| Ethanol (absolut) | 30,0 |
| Wasser | 1,0 |
| Ascorbinsäure | 0,005 |
| HFA-134a | 68,985 |

### Formulierungsbeispiel 11:

| **Bestandteile** | **Konzentration [% w/w]** |
|---|---|
| Tiotropiumjodid | 0,01 |
| Ethanol (absolut) | 40,0 |
| Zitronensäure | 0,004 |
| HFA-227 | 59,986 |

### Formulierungsbeispiel 12:

| **Bestandteile** | **Konzentration [% w/w]** |
|---|---|
| Tiotropiumchlorid | 0,02 |
| Ethanol (absolut) | 25,0 |
| Wasser | 1,0 |
| Zitronensäure | 0,003 |
| HFA-134a | 73,977 |

### Formulierungsbeispiel 13:

| **Bestandteile** | **Konzentration [% w/w]** |
|---|---|
| Tiotropiumchlorid | 0,02 |
| Ethanol (absolut) | 20,0 |
| HCl (aq) 0,01 mol/l | 2,0 |
| HFA-134a | 77,98 |

### Formulierungsbeispiel 14:

| **Bestandteile** | **Konzentration [% w/w]** |
|---|---|
| Tiotropiumchlorid | 0,01 |
| Ethanol (absolut) | 15,0 |
| Wasser | 2,0 |
| Zitronensäure | 0,004 |
| HFA-227 | 82,986 |

### Formulierungsbeispiel 15:

| **Bestandteile** | **Konzentration [% w/w]** |
|---|---|
| Tiotropiumchlorid | 0,01 |
| Ethanol (absolut) | 30,0 |
| Wasser | 1,0 |
| Ascorbinsäure | 0,005 |
| HFA-134a | 68,985 |

### Formulierungsbeispiel 16:

| **Bestandteile** | **Konzentration [% w/w]** |
|---|---|
| Tiotropiumchlorid | 0,01 |
| Ethanol (absolut) | 40,0 |
| Zitronensäure | 0,004 |
| HFA-227 | 59,986 |

### Formulierungsbeispiel 17:

| **Bestandteile** | **Konzentration [% w/w]** |
|---|---|
| Tiotropiumjodid | 0,02 |
| Ethanol (absolut) | 25,0 |
| Wasser | 1,0 |
| Zitronensäure | 0,003 |
| HFA-134a | 73,977 |

### Formulierungsbeispiel 18:

| **Bestandteile** | **Konzentration [% w/w]** |
|---|---|
| Tiotropiumjodid | 0,02 |
| Ethanol (absolut) | 20,0 |
| HCl (aq) 0,01 mol/l | 2,0 |
| HFA-134a | 77,98 |

### Formulierungsbeispiel 19:

| **Bestandteile** | **Konzentration [% w/w]** |
|---|---|
| Tiotropiumjodid | 0,01 |
| Ethanol (absolut) | 15,0 |
| Wasser | 2,0 |
| Zitronensäure | 0,004 |
| HFA-227 | 82,986 |

### B.2.2. Aerosolsuspensionsformulierungen

Die vorliegende Erfindung betrifft ferner Suspensionen der erfindungsgemäßen Tiotropiumsalze Tiotropiumchlorid und Tiotropiumjodid in den Treibgasen HFA 227 und/oder HFA 134a, gegebenenfalls in Mischung mit einem oder mehreren weiteren Treibgasen, vorzugsweise ausgewählt aus der Gruppe bestehend aus Propan, Butan, Pentan, Dimethylether, CHClF₂, CH₂F₂, CF₃CH₃, Isobutan, Isopentan und Neopentan. Erfindungsgemäß bevorzugt sind solche Suspensionen, die als Treibgas nur HFA 227, ein Gemisch aus HFA 227 und HFA 134a oder nur HFA 134a enthalten.

Wird in den erfindungsgemäßen Suspensionsformulierungen ein Gemisch der Treibgase HFA 227 und HFA 134a eingesetzt, so sind die Gewichtsverhältnisse, in denen diese beiden Treibgaskomponenten zum Einsatz gelangen können, frei variabel.

Werden in den erfindungsgemäßen Suspensionsformulierungen neben den Treibgasen HFA 227 und/oder HFA 134a ein oder mehrere weitere Treibgase, ausgewählt aus der Gruppe bestehend aus Propan, Butan, Pentan, Dimethylether, CHClF₂, CH₂F₂, CF₃CH₃, Isobutan, Isopentan und Neopentan eingesetzt, so liegt der Anteil dieser weiteren Treibgaskomponente vorzugsweise unter 50 %, bevorzugt unter 40% besonders bevorzugt unter 30%.

Die erfindungsgemäßen Suspensionen enthalten vorzugsweise eine solche Menge an Tiotropiumchlorid oder -jodid, dass der Anteil an Tiotropiumkation zwischen 0,001 bis 0,8%, erfindungsgemäß bevorzugt zeischen 0,08 bis 0,5%, besonders bevorzugt zwischen 0,2 bis 0,4% beträgt.

Bei den im Rahmen der vorliegenden Erfindung genannten prozentualen Angaben, handelt es sich sofern nicht gegenteilig angegeben stets um Gewichtsprozent.

Gegebenenfalls wird im Rahmen der vorliegenden Erfindung statt des Begriffs Suspension auch der Begriff Suspensionsformulierung verwendet. Beide Begriffe sind im Rahmen der vorliegenden Erfindung als gleichbedeutend anzusehen.

Die erfindungsgemäßem treibgashaltigen Inhalationsaerosole bzw. Suspensionsformulierungen können ferner weitere Bestandteile wie oberflächenaktive Mittel (Tenside, Surfactants), Adjuvantien, Antioxidantien oder Geschmacksmittel enthalten.

Die in den erfindungsgemäßen Suspensionen gegebenenfalls enthaltenen oberflächenaktiven Mittel (Tenside, Surfactants) sind vorzugsweise ausgewählt aus der Gruppe bestehend aus Polysorbat 20, Polysorbat 80, Myvacet 9-45, Myvacet 9-08, Isopropylmyristat, Ölsäure, Propylenglycol, Polyethylenglycol, Brij, Ethyloleat, Glyceryltrioleat Glycerylmonolaurat , Glycerylmonooleat, Glycerylmonosterat, Glycerylmonoricinoleate, Cetylalcohol, Sterylalkohol, Cetylpyridinumchlorid, Blockpolymere, natürliches Öl, Ethanol und Isopropanol. Von den vorstehend genannten Suspensionshilfsstoffen gelangen bevorzugt zur Anwendung Polysorbat 20, Polysorbat 80, Myvacet 9-45, Myvacet 9-08 oder Isopropylmyristat. Besonders bevorzugt gelangen zur Anwendung Myvacet 9-45 oder Isopropylmyristat.

Sofern in den erfindungsgemäßen Suspensionen oberflächenaktive Mittel enthalten sind, werden diese vorzugsweise in einem Anteil von 0,0005 - 1 %, besonders bevorzugt 0,005 - 0,5 % eingesetzt.

Die in den erfindungsgemäßen Suspensionen gegebenenfalls enthaltenen Adjuvantien sind vorzugsweise ausgewählt aus der Gruppe bestehend aus Alanin, Albumin, Ascorbinsäure, Aspartam, Betain, Cystein, Phosphorsäure, Salpetersäure, Salzsäure, Schwefelsäure und Zitronensäure. Besonders bevorzugt gelangen dabei zur Anwendung Ascorbinsäure, Phosphorsäure, Salzsäure oder Zitronensäure, besonders bevorzugt Salzsäure oder Zitronensäure.

Sofern in den erfindungsgemäßen Suspensionen Adjuvantien enthalten sind, werden diese vorzugsweise in einem Anteil von 0,0001-1,0 %, bevorzugt 0,0005-0,1 %, besonders bevorzugt 0,001-0,01 % eingesetzt, wobei ein Anteil von 0,001-0,005 % erfindungsgemäß von besonderer Bedeutung ist.

Die in den erfindungsgemäßen Suspensionen gegebenenfalls enthaltenen Antioxidantien sind vorzugsweise ausgewählt aus der Gruppe bestehend aus Ascorbinsäure, Zitronensäure, Natriumedetat, Editinsäure Tocopherolen, Butylhydroxytoluol, Butylhydroxyanisol und Ascorbylpalmitat, wobei Tocopherole, Butylhydroxytoluol, Butylhydroxyanisol oder Ascorbylpalmitat bevorzugt zum Einsatz gelangen.

Die in den erfindungsgemäßen Suspensionen gegebenenfalls enthaltenen Geschmacksmittel sind vorzugsweise ausgewählt aus der Gruppe bestehend aus Pefferminz, Sacharin, Dentomint, Aspartam und etherischen Ölen (beispielsweise Zimt, Anis, Menthol, Campher), wobei beispielsweise Pefferminz oder Dentomint® besonders bevorzugt sind.

Im Hinblick auf die inhalative Applikation ist es erforderlich, die Wirkstoffe in feinteiliger Form bereitzustellen. Die erfindungsgemäßen Salze Tiotropiumchlorid und -jodid werden dazu entweder gemahlen (mikronisiert) oder über andere technische, im Stand der Technik prinzipiell bekannte Verfahren (beispielsweise Präzipitation, Sprühtrocknung) in feinteiliger Form gewonnen. Verfahren zur Mikronisierung von Wirkstoffen sind im Stand der Technik bekannt. Vorzugsweise weist der Wirkstoff nach Mikronisierung eine mittlere Teilchengröße von 0,5 bis 10µm, bevorzugt von 1 bis 6µm, besonders bevorzugt von 1,5 bis 5µm auf. Vorzugsweise weisen wenigstens 50%, bevorzugt wenigstens 60%, besonders bevorzugt wenigstens 70% der Wirkstoffteilchen eine Teilchengröße auf, die innerhalb der vorstehend genannten Größenbereiche liegt. Besonders bevorzugt liegen wenigstens 80%, höchst bevorzugt wenigstens 90% der Wirkstoffteilchen mit ihrer Partikelgröße in den vorstehend genannten Bereichen.

Ein weiterer Aspekt der vorliegenden Erfindung zielt auf Suspensionen, die lediglich einer der beiden erfindungsgemäßen Wirkstoffe ohne weitere Zusätze enthalten.

Zur Herstellung der erfindungsgemäßen Suspensionen kann nach im Stand der Technik bekannten Verfahren vorgegangen werden. Hierzu werden die Bestandteile der Formulierung mit dem oder den Treibgasen (ggf. bei niedrigen Temperaturen) gemischt und in geeignete Behälter abgefüllt.

Die vorstehend genannten erfindungsgemäßen treibgashaltigen Suspensionen können mittels im Stand der Technik bekannten Inhalatoren (pMDIs = pressurized metered dose inhalers) appliziert werden. Dementsprechend betrifft ein weiterer Aspekt der vorliegenden Erfindung Arzneimittel in Form von wie vorstehend beschriebenen Suspensionen in Verbindung mit einem oder mehreren zur Verabreichung dieser Suspensionen geeigneten Inhalatoren. Ferner betrifft die vorliegende Erfindung Inhalatoren, dadurch gekennzeichnet, daß sie vorstehend beschriebene erfindungsgemäße treibgashaltige Suspensionen enthalten.

Die vorliegende Erfindung betrifft ferner Behälter (Kartuschen), die ausgestattet mit einem geeigneten Ventil in einem geeigneten Inhalator zur Anwendung gelangen können und die eine der vorstehend genannten erfindungsgemäßen treibgashaltigen Suspensionen enthalten. Geeignete Behälter (Kartuschen) und Verfahren zur Abfüllung dieser Kartuschen mit den erfindungsgemäßen treibgashaltigen Suspensionen sind aus dem Stand der Technik bekannt.

Aufgrund der pharmazeutischen Wirksamkeit von Tiotropium betrifft die vorliegende Erfindung ferner die Verwendung der erfindungsgemäßen Suspensionen zur Herstellung eines inhalativ oder nasal applizierbaren Arzneimittels, bevorzugt zur Herstellung eines Arzneimittels zur inhalativen oder nasalen Behandlung von Erkrankungen, in denen Anticholinergika einen therapeutischen Nutzen entfalten können.

Besonders bevorzugt betrifft die vorliegende Erfindung ferner die Verwendung der erfindungsgemäßen Suspensionen zur Herstellung eines Arzneimittels zur inhalativen Behandlung von Atemwegserkrankungen, bevorzugt von Asthma oder COPD.

Die nachfolgenden Beispiele dienen der exemplarischen, weitergehenden Illustration der vorliegenden Erfindung, ohne selbige auf deren Inhalt zu beschränken.

### B.2.1.2 Beispiele für Aerosolsuspensionsformulierungen

Suspensionen enthaltend neben Wirkstoff und Treibgas weitere Bestandteile:

### Formulierungsbeispiel 20:

| **Bestandteile** | **Konzentration [% w/w]** |
|---|---|
| Tiotropiumjodid | 0,02 |
| Isopropylmyristat | 0,30 |
| HFA-227 | 20,00 |
| HFA-134a | 79,68 |

### Formulierungsbeispiel 21:

| **Bestandteile** | **Konzentration [% w/w]** |
|---|---|
| Tiotropiumchlorid | 0,04 |
| Ölsäure | 0,005 |
| HFA-227 | 99,955 |

### Formulierungsbeispiel 22:

| **Bestandteile** | **Konzentration [% w/w]** |
|---|---|
| Tiotropiumjodid | 0,02 |
| Ölsäure | 0,01 |
| HFA-227 | 60,00 |
| HFA-134a | 39,97 |

### Formulierungsbeispiel 23:

| **Bestandteile** | **Konzentration [% w/w]** |
|---|---|
| Tiotropiumjodid | 0,02 |
| Isopropylmyristat | 1,00 |
| HFA-227 | 98,98 |

### Formulierungsbeispiel 24:

| **Bestandteile** | **Konzentration [% w/w]** |
|---|---|
| Tiotropiumchlorid | 0,02 |
| Myvacet 9-45 | 0,3 |
| HFA-227 | 99,68 |

### Formulierungsbeispiel 25:

| **Bestandteile** | **Konzentration [% w/w]** |
|---|---|
| Tiotropiumchlorid | 0,02 |
| Myvacet 9-45 | 0,1 |
| HFA-227 | 60,00 |
| HFA-134a | 39,88 |

### Formulierungsbeispiel 26:

| **Bestandteile** | **Konzentration [% w/w]** |
|---|---|
| Tiotropiumchlorid | 0,04 |
| Polysorbat 80 | 0,04 |
| HFA-227 | 99,92 |

### Formulierungsbeispiel 27:

| **Bestandteile** | **Konzentration [% w/w]** |
|---|---|
| Tiotropiumjodid | 0,01 |
| Polysorbat 20 | 0,20 |
| HFA-227 | 99,78 |

### Formulierungsbeispiel 28:

| **Bestandteile** | **Konzentration [% w/w]** |
|---|---|
| Tiotropiumchlorid | 0,04 |
| Myvacet 9-08 | 01,00 |
| HFA-227 | 98,96 |

Suspensionen enthaltend lediglich Wirkstoff und Treibgas:

### Formulierungsbeispiel 29:

| **Bestandteile** | **Konzentration [% w/w]** |
|---|---|
| Tiotropiumjodid | 0,04 |
| HFA-227 | 80,00 |
| HFA-134a | 19,96 |

### Formulierungsbeispiel 30:

| **Bestandteile** | **Konzentration [% w/w]** |
|---|---|
| Tiotropiumjodid | 0,02 |
| HFA-227 | 60,00 |
| HFA-134a | 39,98 |

### Formulierungsbeispiel 31:

| **Bestandteile** | **Konzentration [% w/w]** |
|---|---|
| Tiotropiumjodid | 0,02 |
| HFA-227 | 99,98 |

### Formulierungsbeispiel 32:

| **Bestandteile** | **Konzentration [% w/w]** |
|---|---|
| Tiotropiumjodid | 0,02 |
| HFA-134a | 99,98 |

### Formulierungsbeispiel 33:

| **Bestandteile** | **Konzentration [% w/w]** |
|---|---|
| Tiotropiumchlorid | 0,02 |
| HFA-227 | 99,98 |

### Formulierungsbeispiel 34:

| **Bestandteile** | **Konzentration [% w/w]** |
|---|---|
| Tiotropiumchlorid | 0,02 |
| HFA-134a | 99,98 |

### Formulierungsbeispiel 35:

| **Bestandteile** | **Konzentration [% w/w]** |
|---|---|
| Tiotropiumchlorid | 0,02 |
| HFA-227 | 20,00 |
| HFA-134a | 79,98 |

### Formulierungsbeispiel 36:

| **Bestandteile** | **Konzentration [% w/w]** |
|---|---|
| Tiotropiumchlorid | 0,04 |
| HFA-227 | 40,00 |
| HFA-134a | 59,96 |

### B.3. Treibgasfreie Inhalationsaerosole

Die neuen Tiotropiumsalze Tiotropiumchlorid oder Tiotropiumjodid können gegebenenfalls auch in Form von treibgasfreien Inhalationsaerosolen appliziert werden.

Zur Applikation dieser treibgasfreien Inhalationsaerosole werden die neuen Tiotropiumsalze Tiotropiumchlorid oder Tiotropiumjodid in Form von Arzneimiitellösungen bereitgestellt.

Dabei kann das Lösungsmittel ausschließlich Wasser sein, oder es ist ein Gemisch aus Wasser und Ethanol. Der relative Anteil an Ethanol gegenüber Wasser ist nicht begrenzt, bevorzugt liegt die maximale Grenze jedoch bei bis 70 Volumenprozent, insbesondere bei bis zu 60 Volumenprozent und besonders bevorzugt bei bis zu 30 Volumenprozent. Die restlichen Volumenprozente werden von Wasser aufgefüllt. Bevorzugtes Lösungsmittel ist Wasser ohne ethanolischen Zusatz.

Die Konzentration der erfindungsgemäßen neuen Tiotropiumsalze Tiotropiumchlorid oder Tiotropiumjodid bezogen auf den Anteil an Tiotropium in der fertigen Arzneimittelzubereitung ist abhängig von dem angestrebten therapeutischen Effekt. Für die Mehrzahl der auf Tiotropium ansprechenden Erkrankungen liegt die Konzentration an Tiotropium zwischen 0,0005 und 5 Gew. %, bevorzugt zwischen 0,001 und 3 Gew.%. Der pH-Wert der erfindungsgemäßen Formulierung liegt zwischen 2,0 und 4,5, bevorzugt zwischen 2,5 und 3,5 und stärker bevorzugt zwischen 2,7 und 3,3 und besonders bevorzugt zwischen 2,7 und 3,2. Am stärksten bevorzugt sind pH-Werte mit einer oberen Grenze von 3,1.

Der pH-Wert wird durch Zugabe von pharmakologisch verträglichen Säuren eingestellt. Beispiele für diesbezüglich bevorzugte anorganische Säuren sind: Salzsäure, Bromwasserstoffsäure, Salpetersäure, Schwefelsäure und/oder Phosphorsäure.

Beispiele für besonders geeignete organische Säuren sind: Ascorbinsäure, Zitronensäure, Äpfelsäure, Weinsäure, Maleinsäure, Bernsteinsäure, Fumarsäure, Essigsäure, Ameisensäure und/oder Propionsäure und andere. Bevorzugte anorganische Säuren sind Salzsäure, Schwefelsäure. Es können auch die Säuren verwendet werden, die bereits mit dem Wirkstoff ein Säureadditionssalz bilden.

Unter den organischen Säuren sind Ascorbinsäure, Fumarsäure und Zitronensäure bevorzugt, wobei Zitronensäure am stärksten bevorzugt ist. Gegebenenfalls können auch Gemische der genannten Säuren eingesetzt werden, insbesondere in Fällen von Säuren, die neben ihren Säuerungseigenschaften auch andere Eigenschaften, z.B. als Geschmackstoffe oder Antioxidantien besitzen, wie beispielsweise Zitronensäure oder Ascorbinsäure.

Als anorganische Säuren wird ausdrücklich Salzsäure genannt.

Gegebenenfalls können auch pharmakologisch verträgliche Basen zum genauen Austitrieren des pH-Wertes eingesetzt werden. Als Basen eignen sich beispielsweise Alkalihydroxide und Alkalicarbonate. Bevorzugtes Alkaliion ist Natrium. Werden solche Basen verwendet, ist darauf zu achten, daß auch die daraus resultierenden Salze, die dann in der fertigen Arzneimittelformulierung enthalten sind, mit der oben genannten Säure pharmakologisch akzeptabel ist.

Erfindungsgemäß kann in der vorliegenden Formulierung auf den Zusatz von Editinsäure (EDTA) oder einem der bekannten Salze davon, Natriumedetat, als Stabilisator oder Komplexbildner verzichtet werden.

Eine andere Ausführungsform beinhaltet Editinsäure und/oder die genannten Salze davon.

In einer solchen bevorzugten Ausführungsform liegt der Gehalt bezogen aufNatriumedetat unter 10 mg / 100 ml. In diesem Fall findet sich ein bevorzugter Bereich zwischen 5 mg/100 ml und kleiner 10 mg/100 ml oder ein anderer zwischen größer 0 und 5 mg/100ml.

In einer anderen Ausführungsform beträgt der Gehalt an Natriumedetat 10 bis zu 30 mg / 100 ml, bevorzugt beträgt er maximal 25 mg/ 100 ml.

In einer bevorzugten Ausführungsform wird auf diesen Zusatz gänzlich verzichtet.

Analoges wie bereits für Natriumedetat ausgeführt, gilt auch für andere vergleichbare Zusatzstoffe, die komplexbildende Eigenschaften aufweisen und anstelle dessen verwendet werden können, wie beispielsweise Nitrilotriessigsäure und deren Salze.

Unter Komplexbildner werden im Rahmen der vorliegenden Erfindung bevorzugt Moleküle verstanden, die in der Lage sind Komplexbindungen einzugehen. Bevorzugt sollen durch diese Verbindungen Kationen, besonders bevorzugt metallische Kationen komplexiert werden.

Der erfindungsgemäßen Formulierung können neben Ethanol auch weitere Co-Solventien und/oder weitere Hilfsstoffe zugesetzt werden.

Bevorzugte Co-Solventien sind solche, die Hydroxylgruppen oder andere polare Gruppen enthalten, beispielsweise Alkohole - insbesondere Isopropylalkohol, Glykole - insbesondere Propylenglykol, Polyethylenglykol, Polypropylenglykol, Glykolether, Glycerol, Polyoxyethylenalkohole und Polyoxyethylen-Fettsäureester, sofern sie nicht bereits das Lösungs- oder Suspensionsmittel sind.

Unter Hilfs- und Zusatzstoffen wird in diesem Zusammenhang jeder pharmakologisch verträgliche und therapeutisch sinnvolle Stoff verstanden, der kein Wirkstoff ist, aber zusammen mit dem Wirkstoff in dem pharmakologisch geeigneten Lösungsmittel formuliert werden kann, um die qualitativen Eigenschaften der Wirkstoffformulierung zu verbessern. Bevorzugt entfalten diese Stoffe keine oder im Kontext mit der angestrebten Therapie keine nennenswerte oder zumindest keine unerwünschte pharmakologische Wirkung. Zu den Hilfs- und Zusatzstoffen zählen z.B. oberflächenaktive Stoffe, wie z.B. Sojalecithin, Ölsäure, Sorbitanester, wie Sorbitantrioleat, Polyvinylpyrrolidon sonstige Stabilisatoren, Komplexbildner, Antioxidantien und/oder Konservierungsstoffe, die die Verwendungsdauer der fertigen Arzneimittelformulierung verlängern, Geschmackstoffe, Vitamine und/oder sonstige dem Stand der Technik bekannte Zusatzstoffe. Zu den Zusatzstoffen zählen auch pharmakologisch unbedenkliche Salze wie beispielsweise Natriumchlorid.

Zu den bevorzugten Hilfsstoffen zählen Antioxidantien, wie beispielsweise Ascorbinsäure, sofern nicht bereits für die Einstellung des pH-Werts verwendet, Vitamin A, Vitamin E, Tocopherole und ähnliche im menschlichen Organismus vorkommende Vitamine oder Provitamine.

Konservierungsstoffe können eingesetzt werden, um die Formulierung vor Kontamination mit pathogenen Keimen zu schützen. Als Konservierungsstoffe eignen sich die dem Stand der Technik bekannten, insbesondere Benzalkoniumchlorid oder Benzoesäure bzw. Benzoate wie Natriumbenzoat in der aus dem Stand der Technik bekannten Konzentration.

Bevorzugte Formulierungen enthalten außer dem Lösungsmittel Wasser und einem der neuen Tiotropiumsalze Tiotropiumchlorid oder Tiotropiumjodid nur noch Benzalkoniumchlorid und Natriumedetat.

In einer anderen bevorzugten Ausführungsform wird auf Natriumedetat verzichtet.

Die erfindungsgemäßen Lösungen werden bevorzugt mittels des Inhalators Respimat^{®} appliziert. Eine weiterentwickelte Ausführungsform dieses Inhalators ist in der WO 97/12687 und deren Figuren 6 offenbart.

### B.3.1. Beispiele für treibgasfreie Inhalationsaerosole

Die nachfolgenden Beispiele dienen der exemplarischen, weitergehenden Illustration der vorliegenden Erfindung, ohne selbige auf deren Inhalt zu beschränken.

### Formulierungsbeispiel 37:

| **Bestandteile** | **Menge** |
|---|---|
| Tiotropiumchlorid | 0,03 g |
| Benzalkonumchlorid | 10 mg |
| Natriumedetat | 10 mg |
| IN HCl (aq) | ad pH 2,9 |
| Wasser | ad 100 g |

### Formulierungsbeispiel 38:

| **Bestandteile** | **Menge** |
|---|---|
| Tiotropiumchlorid | 0,10 g |
| Benzalkonumchlorid | 10 mg |
| Natriumedetat | 25 mg |
| 1N HC1 (aq) | ad pH 3 |
| Wasser | ad 100 g |

### Formulierungsbeispiel 39:

| **Bestandteile** | **Menge** |
|---|---|
| Tiotropiumjodid | 0,05 g |
| Benzalkonumchlorid | 10 mg |
| Natriumedetat | 10 mg |
| 1N HC1 (aq) | ad pH 2,9 |
| Wasser | ad 100 g |

### Formulierungsbeispiel 40:

| **Bestandteile** | **Menge** |
|---|---|
| Tiotropiumjodid | 0,03 g |
| Benzalkonumchlorid | 10 mg |
| Natriumedetat | 10 mg |
| IN HCl (aq) | ad pH 2,9 |
| Wasser | ad 100 g |

### Formulierungsbeispiel 41:

| **Bestandteile** | **Menge** |
|---|---|
| Tiotropiumjodid | 0,10 g |
| Benzalkonumchlorid | 10 mg |
| Natriumedetat | 25 mg |
| IN HCl (aq) | ad pH 3 |
| Wasser | ad 100 g |

### Formulierungsbeispiel 42:

| **Bestandteile** | **Menge** |
|---|---|
| Tiotropiumchlorid | 0,04 g |
| Benzalkonumchlorid | 10 mg |
| Natriumedetat | 10 mg |
| IN HCl (aq) | ad pH 2,9 |
| Wasser | ad 100 g |

## Patentansprüche

1. Kristallines Tiotropiumjodid, gegebenenfalls in Form seiner Solvate oder Hydrate.

2. Kristallines Tiotropiumjodid nach Anspruch 1, welches **dadurch gekennzeichnet ist, dass** es im Röntgenpulverdiagramm unter anderen die charakteristischen Werte d= 6,30 Å; 5,19 Å; 4,47 Å; 4,11 Ä und 3,55 Ä aufweist.

3. Verwendung eines Tiotropiumsalzes nach einem der Ansprüche 1-2 zur Herstellung eines Arzneimittels zur Behandlung von Atemwegserkrankungen, insbesondere zur Behandlung von COPD (chronic obstructive pulmonary disease = chronisch obstruktive Lungenerkrankung) und Asthma.

4. Arzneimittel, **dadurch gekennzeichnet dass** es ein Tiotropiumsalz nach einem der Ansprüche 1-2 enthält.

5. Arzneimittel nach Anspruch 4, **dadurch gekennzeichnet, daß** es ein Inhalationspulver ist, welches neben dem Tiotropiumsalz einen oder mehrere geeignete physiologisch unbedenkliche Hilfsstoffe ausgewählt aus der Gruppe der Monosaccharide, der Disaccharide, der Oligo- und Polysaccharide, der Polyalkohole, der Cyclodextrine, der Aminosäuren oder auch der Salze oder Mischungen dieser Hilfsstoffe miteinander enthält.

6. Arzneimittel nach Anspruch 5, **dadurch gekennzeichnet, dass** der Hilfsstoff ausgewählt ist aus der Gruppe bestehend aus Glucose, Fructose, Arabinose, Lactose, Saccharose, Maltose, Trehalose, Dextrane, Dextrine, Maltodextrin, Stärke, Cellulose, Sorbit, Mannit, Xylit, α-Cyclodextrin, β-Cyclodextrin, χ-Cyclodextrin, Methyl-β-Cyclodextrin, Hydroxypropyl-β-Cyclodextrin, Argininhydrochlorid, Natriumchlorid oder Calciumcarbonat, oder Mischungen davon.

7. Arzneimittel nach Anspruch 4, **dadurch gekennzeichnet, daß** es sich um ein treibgashaltiges Inhalationsaerosol handelt, welches das Tiotropiumsalz in gelöster oder dispergierter Form enthält.

8. Arzneimittel nach Anspruch 4, **dadurch gekennzeichnet, daß** es sich um eine treibgasfreie Inhalationslösung oder -suspension handelt, die als Lösemittel Wasser, Ethanol oder ein Gemisch aus Wasser und Ethanol enthält.
